# EUROPEAN PATENT SPECIFICATION

(11) **EP 1 411 814 B1**
(45) Date of publication and mention of the grant of the patent: **01.03.2006**
(21) Application number: 01992521.3
(22) Date of filing: 01.11.2001
(51) Int. Cl.: A61B 1/00, A61B 17/43, A61B 17/435

(54) **DEVICE FOR TRANS-CERVICAL ARTIFICIAL INSEMINATION AND EMBRYO TRANSFER**
VORRICHTUNG FÜR TRANSZERVIKALE ARTIFIZIELLE INSEMINATION UND EMBRYONENTRANSFER
DISPOSITIF D'INSEMINATION ARTIFICIELLE TRANSCERVICALE ET DE TRANSFERT D'EMBRYON

(30) Priority: 03.11.2000 US 705489; 07.09.2001 US 318015 P
(43) Date of publication of application: 28.04.2004
(73) Proprietor: Continental Plastic Corp., Delavan, WI 53112-0902 (US); Christine, Robert R., Bethel, MO 63434 (US); Schoenbeck, Ricky A., Walworth, WI 53184 (US); Hladky, Donald F., Janesville, WI 53545 (US)
(72) Inventor: CHRISTINE, Robert, R., Bethel, MO 63434 (US); SCHOENBECK, Ricky, A., Walworth, WI 53184 (US); HLADKY, Donald, F., Janesville, WI 53545 (US)
(74) Representative: Bauer, Dirk
(86) International application number: PCT/US2001/044917
(87) International publication number: WO 2002/035982

(56) References cited:
- EP-A1- 0 189 702
- EP-A2- 0 605 406
- FR-A- 2 432 866
- US-A- 4 457 313
- US-A- 4 474 576
- US-A- 5 496 272
- US-A- 5 916 144

## Description

### 1. Cross-Reference to Related Applications

This application claims priority based on US Serial No. 09/705,489 and US Provisional Patent Application No. 60/318,815.

### 2. Field of Invention

The present invention relates in general to the field of artificial insemination of mammals. More particularly, the present invention relates to an apparatus and a method useful for non-surgical embryo transfer and artificial insemination of mammals. Specifically, a preferred embodiment of the present invention relates to transfer of fluid medium comprising semen, a fluid medium containing embryos or some medicinal fluid into the uterus of a female mammal such as a sow.

### 3. Discussion of the Related Art

In recent years, effective application of artificial impregnation including artificial insemination and non-surgical embryo transfer has established a proven method for improving the production of domestic livestock. Generally, such techniques enhanced the ability to selectively breed a single genetically superior male for production traits with a large number of females. Selective breeding of course allows for livestock with improved genetic traits for production. Artificial insemination techniques also decrease the chance of diseases and physical injury that can be associated with the natural breeding process. As a result of these and other advantages, the use of artificial insemination and non-surgical embryo transfer have become a widespread technique in the management of many species of domestic livestock. One of the non-surgical embryo transfer systems described in the prior art, involves inserting a tubular instrument into the cervix of a recipient female, and then depositing 10-12 milliliters of liquid medium containing embryos into and through the instrument, the objective being to deposit the embryos in the uterus. However, other procedures have several drawbacks. First, there is no way to determine whether the instrument has been inserted far enough into the cervix so that its forward end is adjacent to the body of the uterus. As a result, instances where the forward end of the instrument remains lodged within the cervix, the embryos may never reach the uterus to initiate pregnancy, and thus pregnancy rate may be reduced. As a result of the aforementioned problems, the pregnancy rate or liter size in embryo-transfer may be reduced. This results in annual monetary losses due to the cost of maintaining the non-pregnant recipient animals.

Other conventional artificial insemination (AI) techniques in the industry for some species may result in reduced pregnancy rate or litter size because not enough sperm cells were deposited into the uterus. To compensate for this and to maximize pregnancy rate or liter size, larger numbers of sperm cells are introduced than may be necessary if the entire insemination dose was deposited into the uterus. This is also due to the difficulty associated with passing a conventional straight AI device through the cervix of some species. The reason the passageway of the cervix is difficult to navigate in most mammalian species is that the inside of the cervix has ridged folds of tissue which block straight entry. These folds need to be circumvented to penetrate the cervical passageway and reach the uterine body. Therefore, there is a need for an improved system for affecting the non-surgical transfer of embryos into recipient animals and artificial insemination, particularly those species having a cervix of the type, which is difficult to navigate, such as swine, sheep, and goats.

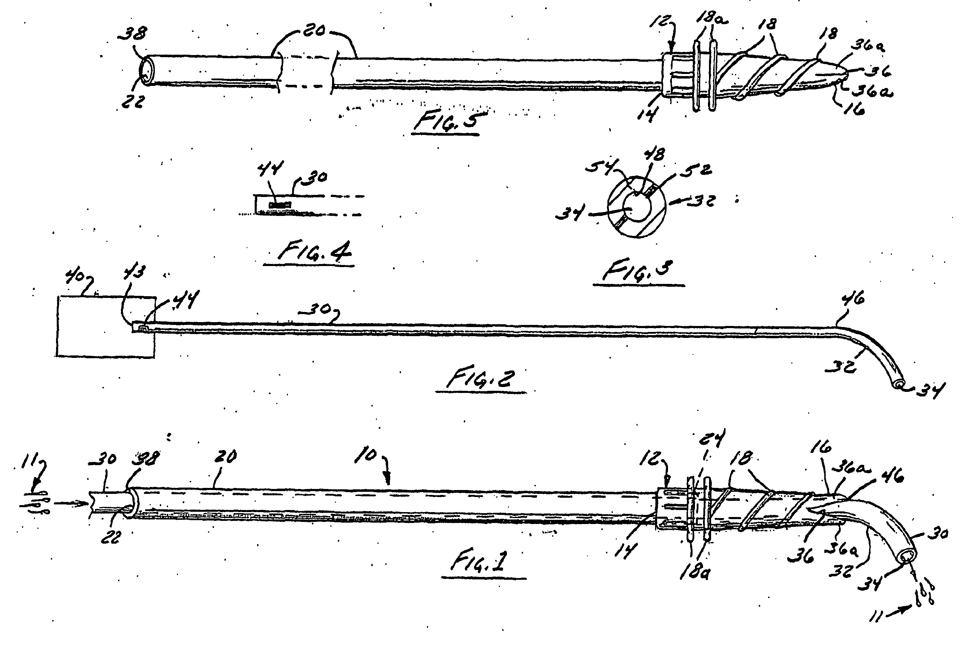

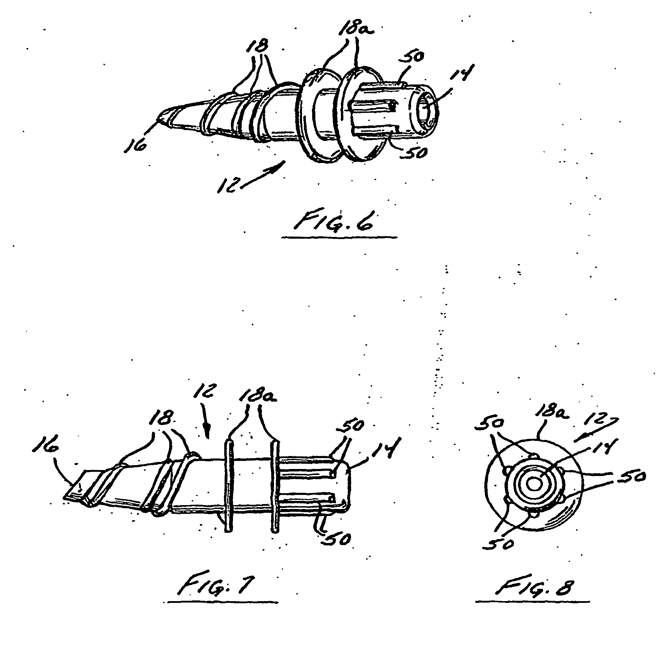

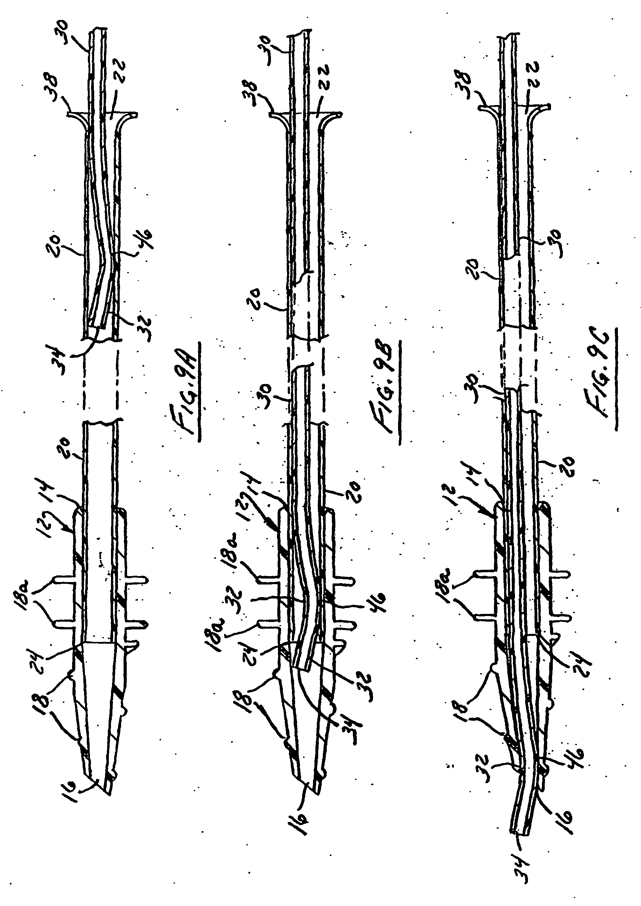

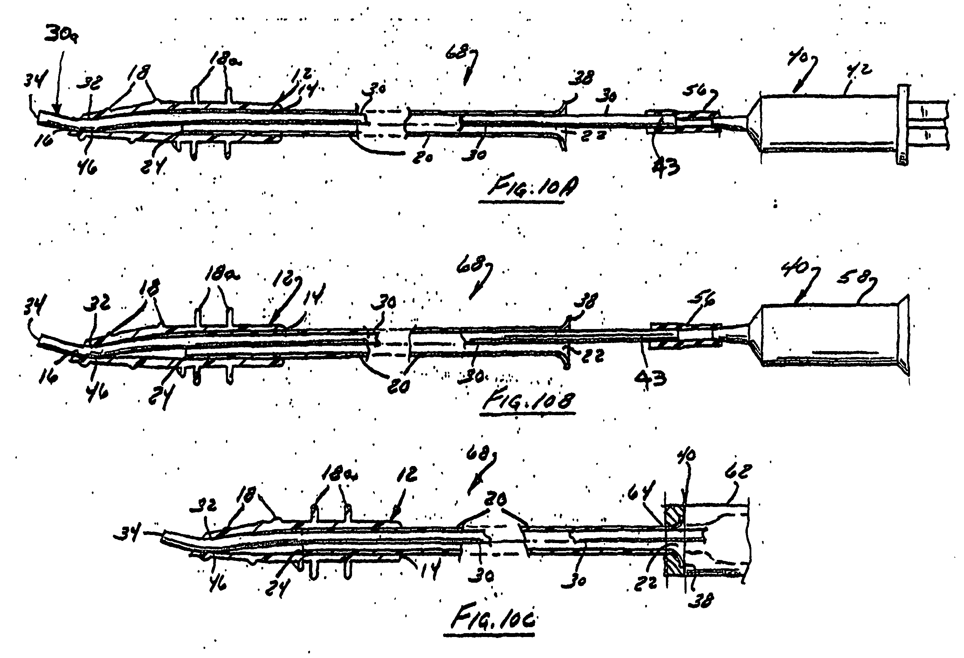

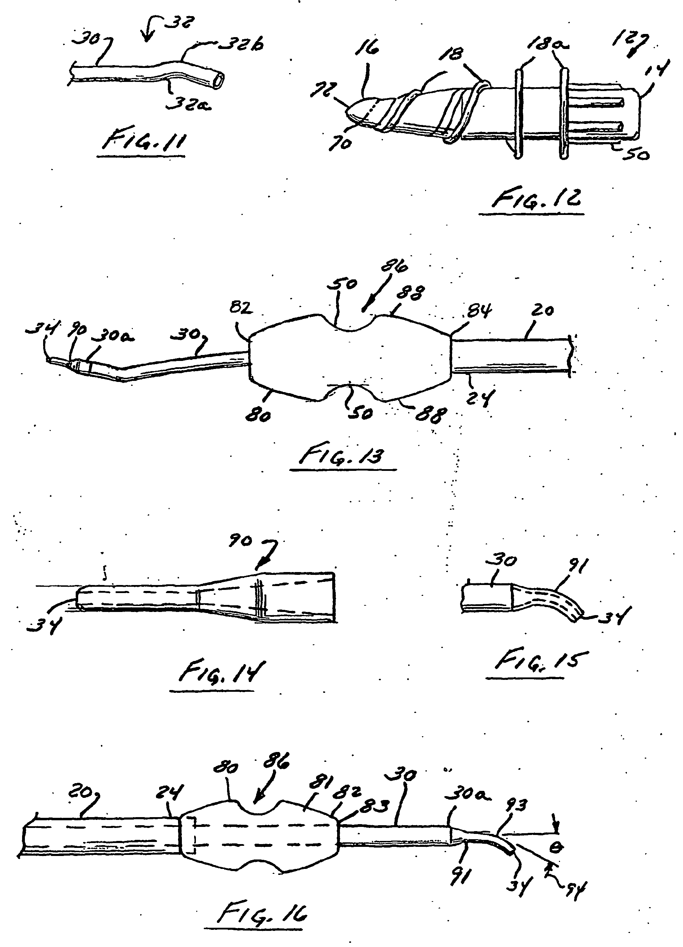

FR 2 432 866 to Cassou at page 2, line 10-page 4, line 25 and Figs. 1-6 shows an artificial insemination gun having a steel tubular body 1 and an attached plastic tubular tip 11 which is screwed into threads 7. A plunger rod with a knob 10 moves semen through the tubular body 1. As best shown in Fig. 3, this tip 11 is shown in a bent position. The tip 11 does not move once it is screwed into place. The relevant content and subject matter of independent claim 1 which is known from document FR 2 432 866 is a bent tubular tip 11 for artificial insemination gun with a tubular body 1.

### SUMMARY AND OBJECTIVES OF THE INVENTION

The present invention relates to an apparatus and a method useful for non-surgical impregnation of mammals. Specifically, a preferred embodiment for the present invention relates to transfer of fluid medium comprising semen or a fluid medium containing embryos into the uterus of an animal.

In accordance with one aspect of the invention, an apparatus for depositing semen into a uterus of a mammal includes a conical chamber that has a plurality of perforations. An exterior spiral formation of the chamber is configured for penetrating the spiral cervical passageway, which provides the entrance to the uterine body. A sheath having a frusto-conical rearward end extends axially from an aft end of the conical chamber. A fluid receptacle or semen or embryo packaging unit is coupled to a rearward end of the sheath. A tubular depositing chamber extends axially from the conical chamber to a position beyond a fore end of the conical chamber. The tubular depositing chamber has an end that has an aperture to permit the flow of semen or embryos out of the depositing chamber and into the uterus. An embryo or semen packaging unit is coupled to the rearward end of the depositing chamber.

In accordance with another aspect of the invention, a method of depositing semen or embryos into the uterus of a mammal includes the steps of: a) inserting a conical chamber having a fore end and an exterior spiral formation into cervix of a mammal, b) securing the conical chamber within walls of the cervix, c) projecting a depositing chamber through an interior portion of the conical chamber, d) moving the depositing chamber transcervically to a semen or embryo release position for release of semen or embryos in the uterus, and e) securing a semen or embryo packaging unit to the depositing member to deposit semen or embryos in the uterus.

Another benefit of the present invention is that for artificial insemination directly into the uterus of some species, the insemination time is reduced. Thus, labor costs are minimized. Still another benefit is that the device is inserted while the animal is standing so breeders can safely more quickly delivering the fluid to a multitude of animals and thus be more efficient.

These and other aspects and objects of the present invention will be better appreciated and understood when considered in conjunction with the following description and the accompanying drawings. It should be understood, however, that the following description, while indicating preferred embodiments of the present invention, is given by way of illustration and not of limitation. Many changes and modifications may be made within the scope of the present invention without departing from the spirit thereof, and the invention includes all such modifications.

### BRIEF DESCRIPTION OF THE DRAWINGS

A clear conception of the advantages and features constituting the present invention, and of the construction and operation of typical mechanisms provided with the present invention, will become more readily apparent by referring to the exemplary, and therefore non-limiting, embodiments illustrated in the drawing accompanying and forming a part of this specification, wherein like reference numerals designate the same elements in the several views, and in which:
Fig. 1 is an elevation view of an apparatus for depositing a medium in a uterus in accordance with the present invention;
Fig. 2 corresponds to Fig 1 and illustrates a tubular depositing chamber and or embryo or semen packaging unit in accordance with the present invention;
Fig. 3 is an end view of the tubular depositing chamber of Fig. 2 showing a raised curvilinear portion thereof;
Fig.4 corresponds to Fig 2 and illustrates a visual marker located on the tubular depositing chamber;
Fig. 5 corresponds to Fig 1 and illustrates a sheath extending axially from an aft end of a conical chamber;
Fig. 6 corresponds to Fig 1 and shows a perspective view of the conical chamber;
Fig. 7 is a plan view of the conical chamber;
Fig. 8 is An end view of Fig. 7;
Fig. 9A corresponds to Fig. 1 with portions broken away to illustrate insertion of the tubular depositing chamber into the sheath;
Fig. 9B corresponds to Fig 9A and illustrates further insertion of the tubular depositing chamber into the sheath;
Fig. 9C corresponds to Fig. 9A and illustrates complete insertion of the tubular depositing chamber through the sheath;
Fig. 10A corresponds to Fig 1 being broken away to illustrate a first preferred embodiment of a complete artificial insemination device using a syringe;
Fig 10B corresponds to Fig. 1 being broken away to illustrate a second preferred embodiment of complete artificial insemination device using a semen packaging plastic tube;
Fig. 10C corresponds to Fig. 1 being broken away to illustrate a third preferred embodiment of a complete artificial insemination device using a plastic bag.
Fig. 11 shows another embodiment of the tubular depositing chamber.
Fig. 12 shows yet another embodiment of the conical chamber
Fig. 13 is a side view of another embodiment of a device of the invention;
Fig. 14 is magnified side view of the tip of Fig. 13 showing the inner configuration is shadow;
Fig. 15 is a magnified side view of another embodiment showing a arched tip configuration; and
Fig. 16 is a side view of another embodiment of a device of the invention showing the inner configuration of the device in shadow.

In describing the preferred embodiment of the invention, which is illustrated in the drawings, specific terminology will be resorted to for the sake of clarity. However, it is not intended that the invention be limited to the specific terms so selected and it is to be understood that each specific term includes a technical equivalents which operate in a similar manner to accomplish a similar purpose. For example, the word "connected" or terms similar thereto are often used. They are not limited to direct connection but include connection through other elements where such connection is recognized as being equivalent by those skilled in the art.

### DETAILED DESCRIPTION OF PREFERRED EMBODIMENTS

### 1. Resume

The present invention and the various features and advantages details thereof are explained more fully with reference to the non-limiting embodiments described in detail in the following description.

Fig. 1 shows an artificial insemination device or apparatus 10 and its components for depositing of semen or other medium 11 into a uterus of a mammal (not shown). The apparatus 10 comprises a sheath 20, a tubular depositing chamber 30, and a conical chamber 12 to be engaged with one another to transfer semen or other fluid medium from a receptacle or packaging unit 40 (Fig. 2) into the uterus of the mammal. The conical chamber 12 has an aft end 14, and a fore end 16, and an exterior spiral formation 18 configured for penetrating the passageway of a cervix. The fluid 11 could also contain live embryos or some medicine. Two circular sealing rings 18a provide a seal to prevent the fluid from leaking out of the cervix.

Fig. 2 shows the tubular depositing chamber 30 having an arcing end 32 for extending axially from the conical chamber 12 to a position beyond the fore end 16 of the conical chamber 12 as can be seen in Fig. 1. The arcing end 32 includes a flat portion 46 that is configured to abut a distal wall of the uterus to stop further insertion of the depositing chamber 30. As best seen in Fig. 1, the depositing chamber 30 is sized to be slidably inserted from arcing end 32 into the rear end 22 of the sheath 20. The length of tubular depositing chamber 30 is considerably longer than the sheath 20 so that, when fully inserted into the apparatus 10, the depositing chamber 30 projects beyond the forward end 24 of the sheath 20. By way of example, the sheath 20 may have an overall length of 53.34 cm (21 inches) and a diameter of 635 cm (0.25 in.). Preferably, the depositing chamber 30 has an overall length of 71.12 cm (28 in.) and a diameter of 23 cm (0.09 in.). These device dimensions are for an embodiment for a sow. Dimensions for other species will be different.

An adapter 56 is connected to the rearward most end 43 of the depositing chamber 30 as shown in Fig. 10A. The adapter 56 may serve as a handle for the depositing chamber 30 because the adapter 56 may be grasped to manipulate the depositing chamber 30 by rotating it about the latitudinal axis and moving it forward and rearward relative to the sheath 20 inside the cervix.

Fig. 3 shows the arcing end 32 of the depositing chamber 30 has an aperture 34 with a raised curvilinear portion 54, which extends from an inner radius 48 to an outer radius 52. The surface of the portion 54 may be polished to smooth potentially rough edges.

As best viewed in Fig. 4, a visual marker 44 is located on the exterior surface of the depositing chamber 30 to indicate a relative orientation of the arcing end 32 of the depositing chamber 30.

Fig. 5 shows a portion of the artificial insemination apparatus 10 in which the sheath 20 and the conical chamber 12 are engaged with one another. The conical chamber 12 includes aft and fore ends 14, 16 and the exterior spiral formation 18 is configured for penetrating the cervical passageway. The fore end 16 may be truncated as shown in Fig. 7. Alternatively, the aft end 14 of the conical chamber 12 may include a plurality of perforations 36 and flaps 36a extending laterally from the fore end 16 of the conical chamber 12 as best shown in Fig. 5. The flaps 36a are of sufficient length to encase the arcing end 32 of the depositing chamber 30 within an interior space of the conical chamber 12. The conical chamber 12 is preferably made of a flexible poly vinyl chloride (PVC) material. The exterior spiral formation 18 allows penetrating easily into the cervix of the mammal.

As best shown in the embodiment of Fig. 7, the conical chamber 12 may have lands 50, which aid during assembly. The lands 50 also provide added friction for gloved hands when pushing inserting the apparatus 10 into the reproductive tract. Figs. 9A through 9C illustrate the insertion of the tubular depositing chamber 30 into the sheath 20 of the artificial insemination apparatus 10. Referring to Fig.9A, the arcing end 32 of the tubular depositing chamber is inserted into the rearward end 22 of the sheath 20 and travels through the sheath 20 until it reaches the conical chamber 12 that is connected (preferably by a friction fit or glued) to the forward end 24 of the sheath 20 as shown in Figs. 9A-C. When the tubular depositing chamber 30 is pushed through the sheath 20, the arcing end 32 of the tubular depositing chamber 30 projects outside of the conical chamber 12 as best shown in Fig. 9C.

Figs. 10A through 10C illustrate the operation of the artificial insemination apparatus 10 using different containers or plastic embryo or semen packaging units. Specifically, Fig. 10A shows an apparatus 68 for depositing a medium 11 having the apparatus 68 in communication with a syringe 42. Fig. 10B shows an apparatus 68 having plastic semen packaging tube 58 such as a container which can be secured to adapter 56 for transferring semen or other media 11 into the depositing chamber 30. By moving the depositing chamber 30 to the semen release position, the medium 11 is discharged into the uterus.

Fig. 10C shows that the receptacle 40 may be a plastic bag 62, which has an opening 64 configured to communicate with the rearward end 22 of the sheath 20 to transfer semen or other media to the uterus. The frusto-conical shape 38 of the rear end 22 allows for a tight fitting of the plastic bag 62 to the apparatus 10.

The device 10 is preferably made of plastic materials. The chamber 12 is made up of a more flexible structure than the sheath 20 or the chamber 30. These materials are better than the stainless steel of prior art devices as they move efficiently and more effectively. The plastic devices can be more easily disposed after use or recycled. They are also more inexpensive to produce. Finally, the more flexible parts make the process more comfortable for the animals and as such there is no need to drug the animal.

As shown in Figure 11, another embodiment has an arcing end 32 having a first bend 32a and a second bend 32b. This "double bend" structure can be used to better maneuver the depositing chamber 30 through the cervical cavity. This feature also reduces the space, from the left to the right extreme, the depositing chamber 30 would occupy in the cervix. Therefore, the "double bend" arcing end 32 will slip through the spiral conical chamber 12 easier and it should pass through the cervix easier, especially the cervix of gilts, which may be smaller and narrower.

As shown in Fig. 12, in one embodiment the conical chamber 12 has a completely closed fore end 16 with at least one perforation 70. The end 16 may be completely sealed for added healthy and safety reasons such as for keeping the enclosed depositing chamber 30 in a sterile environment until it is ready for use. When ready, the operator will break through the perforated tip 72 and the sterile arcing end 32 of the depositing chamber 30 is then pushed past the perforated end 72 of the chamber 12 and through the cervix into the uterus. In one embodiment having multiple perforations, like the one shown in Fig. 5, the chamber 30 may be used to break through the perforations of the conical chamber 12. However, in that embodiment, perforations do not literally break away.

Referring to Figs. 13-16, in these embodiments, the apparatus may consist of a sheath 20, an inner tubular depositing chamber 30, an outer chamber 80 having a fore end 82, an aft end 84, and a ribbed exterior formation 86 configured for traversing a passageway of a cervix. The ribbed configuration 86 is preferably comprised of two or more ribs 88 with grooves or lands 50 between the ribs. The sheath 20 has a forward end 24 and a rearward end (not shown) extending axially from the aft end 84 of the outer chamber 80. Specifically, the outer chamber 80 may be constructed of a soft, pliable foam having twin-tapered ends as shown.

The outer chamber 80 and the exterior ribbed formation 86 are dimensioned and configured for traversing the passageway of a cervix of a mammal, such as a sow. The outer chamber 80 has a wall with a smooth inner surface 81 and a ribbed outer surface 86. The outer chamber 80 is preferably affixed to the sheath 20 by an adhesive such as epoxy glue to prevent the outer chamber 80 from becoming disengaged while in the uterine tract. In one preferred embodiment the outer chamber 80 and sheath 20 are integrally molded from a single piece of plastic.

The inner tubular depositing chamber 30 preferably has an end extending axially from the outer chamber 80 to a position beyond the fore end 82 of the outer chamber 80. The outmost end of the fore end 82 has an aperture 83. Preferably, the tubular depositing chamber 30 in this embodiment narrows in diameter from a rear end (not shown) to the aperture 34 at a front end 30a of the depositing chamber 30 as best shown in Fig. 14. As shown in the embodiment of Fig. 13, the depositing chamber front end 30a includes a tip 90 integral with the depositing chamber 30. In this embodiment, the tip 90 is attached to the bent depositing tubular chamber 30 as shown.

In one embodiment best shown in Figs. 15 and 16, the tip 90 is an arched tip 91 is preferably constructed of a hard plastic. An outside diameter of the tip is preferably narrow enough for the successful transcervical insemination of a gilt while the diameter of the aperture 34 therethrough is wide enough to allow unencumbered passage of boar semen. The arched tip 91 may be a single bend 93 in one embodiment (as shown) or multiple bends in other embodiments. In this embodiment, the depositing tubular chamber is preferably straight although it is foreseeable that a bent depositing chamber 30 would be desirable in combination with an arched tip 91 to give the combination a double bend configuration.

In the embodiment shown in Figs. 15 and 16, the tip 90 is permanently bent at an angle 94 less than 45 degrees from a central axis 95 of the outer chamber 80. In the preferred embodiment, the angle 94 is between 18 and 30 degrees from the central axis 95. If arc is greater than 45 degrees, it is difficult to pass the device through the uterus of a gilt. The arched tip 91 is rigid and preferably has a polished end to prevent it from cutting the uterine wall lining. The arched tip 91 may also have a separate, rounded end which is also preferably smooth. The tip 90 is preferably a distinct member and is affixed to the inner tube 30 by an adhesive.

The inner depositing chamber 30 may be constructed of a flexible plastic such as polyethylene while the rigid arched tip 91 may be constructed from polyvinyl chloride or a high density, more rigid polyethylene.

While the arched tip 91 may constitute a separate member affixed to the inner tube 30, in one preferred embodiment the tip 90 is completely integral with the inner tube 30 and may be molded from a singular piece of extruded tubing. The tip 90 in this embodiment may also have an aperture 34 therethrough with a gradually narrowing frusto-conical inside diameter relative to the rear end of the tubular chamber 30 as best shown in Fig. 15.

### 2. In Use and Operation

When the device is in use with mammals, it can be used to deposit semen, embryos, medicines or other fluids. The animal need not be sedated and can remain standing during the depositing process unlike during most prior art methods. Specifically, the method of depositing fluid into a mammal includes inserting the forward end of the apparatus 10 (i.e. the conical chamber 12) first into a cervix of a uterus of a mammal. The conical-shaped chamber 12 is manipulated through the cervix by turning the connected sheath 20 about an axis and working the apparatus 10 toward the uterus.

This process is made easier by the screw-like, exterior spiral formation 18 of the conical chamber 12. The spiral exterior 18 also helps the operator stimulate the animal on the way to the uterus. Once the conical chamber 12 is in the uterus, it is secured within walls of the cervix by a slight rearward tug of the sheath 20 by the operator. Once secured by the operator, the depositing member or chamber 30 having the arcing end 32 is inserted through the sheath 20. The arcing end 32 is pushed inwardly until it passes through an interior portion of the conical chamber 12. Once the arcing end 32 passes out the depositing chamber 30, it is moved along until the operator experiences substantial resistance to further movement. In one embodiment, the operator can more easily determine the location of this release position by referencing a mark that appears on the shaft of the depositing chamber 30.

Once the operator reaches the fluid release position, a fluid receptacle 40 containing semen or other fluid is then attached to the rear end of depositing member 30. The operator lifts the attached receptacle 40 to a point where gravity helps the fluid move downward into the cavity of the depositing chamber 30 and past the conical chamber 12 to deposit the semen or fluid into the uterus.

The individual components described herein need not necessarily be formed in the disclosed shapes, or assembled in the disclosed configuration, but could be provided in virtually any shape, and assembled in virtually any configuration. For example, although the sheath and tubular depositing chamber could be used separately with minor success, combining them increases the ability to transverse the cervical passageway to the uterus.

Further, although the conical chamber is described herein as a physically separate module, it is apparent that it may be more fully integrated into the sheath. Furthermore, all disclosed features of each disclosed embodiment could be combined with, or substituted for, the disclosed features of every other disclosed embodiment except where such features are mutually exclusive. It is intended that the appended claims cover all such additions, modifications and rearrangements. Expedient embodiments of the present invention are differentiated by the appended sub claims.

## Claims

1. An apparatus (10) for depositing media (11) into a uterus of a mammal, comprising:
a chamber (12) having a conical fore end (16), an aft end (14), and configured for traversing the passageway of a cervix;
a sheath (20) having a forward end (24) and a rearward end (22), wherein said forward end extends axially from the aft end (14) of the chamber (12); and
a slidable tubular depositing chamber (30) having an arcing end (32) for extending axially from the conical chamber (12) to a position beyond the fore end (16) of the conical chamber, wherein the arcing end (32) has an aperture (34) at the fore end.

2. The apparatus of claim 1, **characterized in that**
the conical fore end (16) of the chamber (12) is comprised of a flexible poly vinyl chloride (PVC) material, and comprises a plurality of perforations (36), which extend laterally toward the fore end of the conical chamber, and are adapted to be of sufficient length to allow the arcing end (32) to recess within;
the depositing chamber (30) has a visual marker (44) situated on an exterior surface of the depositing chamber to indicate a relative orientation of the arcing end of the depositing chamber;
the arcing end (32) of the depositing chamber (30) further comprises a flat portion (46), the flat portion (46) configured to abut a distal wall of the uterus to retard further insertion of the depositing chamber; and
further comprising a fluid receptacle (40) attached to the rearward end (22) of the sheath (20) and wherein the rearward end of the sheath further comprises a fiusto-conical shape (38) configured to be inserted into the receptacle (40).

3. The apparatus of claim 1 or 2, **characterized in that** the aperture (34) of the arcing end (32) has an inner radius (48) and an outer radius (52).

4. The apparatus of claim 3 **characterized in that** it comprises a raised curvilinear portion (54) extending from the inner radius (48) to the outer radius (52).

5. The apparatus of claim 1 **characterized in that**:
the conical chamber (12) includes an exterior spiral formation (18) configured for traversing the cervical passageway;
that the sheath (20) has a frusto-conical rearward end (38) extending axially from an aft end of the conical chamber;
that the arcing end (32) of the depositing chamber (30) has a double bend (32a, 32b) and aperture (34) to permit the flow of semen out of the depositing chamber (30) and into the uterus; and
that an embryo or semen packaging unit (40) is coupled to the depositing chamber at the rearward end (43) of the sheath (20) with an adapter (56).

6. The apparatus as in any of the preceding claims, **characterized in that** a plurality of perforations (36) extend laterally to create flaps (36a) from the fore end (16) of the conical chamber (12) within an interior space of the conical chamber (12).

7. The apparatus of claim 1 **characterized in that**:
the chamber 12 is an outer chamber (80) having a fore end, an aft end, and a ribbed exterior formation (86) configured for traversing a passageway of a cervix.

8. The apparatus as in any of claims 1-4 or 7, **characterized in that** the tubular depositing chamber (30) narrows in diameter from the rearward most end (43) to the aperture (34) at the front end (30a) of the depositing chamber (30); and
that the end of the tubular depositing chamber (30) comprises a bent tip (91) integral with the depositing chamber (30).

9. The apparatus as in any of claims 1-4 ,or 7, **characterized in that** it comprises
a bent tip (93) that bends at an angle (94) between 18 and 30 degrees from a central axis of an outer chamber (80).

## Patentansprüche

1. Vorrichtung (10) zum Einbringen von Medien (11) in den Uterus eines Säugetieres, umfassend:
eine Kammer (12) mit einem kegelförmigen Vorderende (16) und einem Hinterende (14), die so konfiguriert ist, dass sie den Durchgang eines Gebärmutterhalses durchquert;
eine Scheide (20) mit einem vorderen Ende (24) und einem hinteren Ende (22), wobei sich das vordere Ende axial vom Hinterende (14) der Kammer (12) erstreckt; und
eine verschiebbare rohrförmige Applikationskammer (30) mit einem bogenförmigen Ende (32), um sich axial von der kegelförmigen Kammer (12) bis zu einer Position außerhalb des Vorderendes (16) der kegelförmigen Kammer zu erstrecken, wobei das bogenförmige Ende (32) am Vorderende eine Öffnung (34) aufweist.

2. Vorrichtung nach Anspruch 1, **dadurch gekennzeichnet, dass**
das kegelförmige Vorderende (16) der Kammer (12) aus einem biegsamen Polyvinylchlorid-Material (PVC) gebildet wird und eine Vielzahl von Perforationen (36) umfasst, die sich seitlich zum Vorderende der kegelförmigen Kammer hin erstrecken und so angepasst sind, dass sie eine ausreichende Länge aufweisen, um es dem bogenförmigen Ende (32) zu erlauben, sich darin zu versenken;
die Applikationskammer (30) eine visuelle Markierung (44) aufweist, die an einer Außenfläche der Applikationskammer angebracht ist, um eine relative Ausrichtung des bogenförmigen Endes der Applikationskammer anzuzeigen;
das bogenförmige Ende (32) der Applikationskammer (30) darüber hinaus einen flachen Abschnitt (46) umfasst, wobei der flache Abschnitt (46) so konfiguriert ist, dass er an eine distale Wand des Uterus stößt, um ein weiteres Einführen der Applikationskammer zu verzögern; und
darüber hinaus ein Flüssigkeitsbehälter (40) vorgesehen ist, der am hinteren Ende (22) der Scheide (20) angebracht ist, wobei das hintere Ende der Scheide darüber hinaus eine kegelstumpfförmige Form (38) umfasst, die konfiguriert ist, um in den Behälter (40) eingeführt zu werden.

3. Vorrichtung nach Anspruch 1 oder 2, **dadurch gekennzeichnet, dass** die Öffnung (34) des bogenförmigen Endes (32) einen inneren Radius (48) und einen äußeren Radius (52) aufweist.

4. Vorrichtung nach Anspruch 3, **dadurch gekennzeichnet, dass** sie einen erhabenen krummlinigen Abschnitt (54) umfasst, der sich vom inneren Radius (48) zum äußeren Radius (52) erstreckt.

5. Vorrichtung nach Anspruch 1, **dadurch gekennzeichnet, dass**
die kegelförmige Kammer (12) eine äußere spiralförmige Struktur (18) umfasst, die so konfiguriert ist, dass sie den Durchgang eines Gebärmutterhalses durchquert;
die Scheide (20) ein kegelstumpfförmiges hinteres Ende (38) aufweist, das sich axial von einem Hinterende der kegelförmigen Kammer erstreckt,
das bogenförmige Ende (32) der Applikationskammer (30) eine doppelte Biegung (32a, 32b) und eine Öffnung (34) aufweist, um das Herausfließen von Samen aus der Applikationskammer (30) und in den Uterus zu ermöglichen; und
eine Embryo- oder Samenverpackungseinheit (40) mit der Applikationskammer am hinteren Ende (43) der Scheide (20) mittels eines Adapters (56) verbunden ist.

6. Vorrichtung nach einem der vorhergehenden Ansprüche, **dadurch gekennzeichnet, dass** sich seitlich eine Vielzahl von Perforationen (36) erstreckt, um Klappen (36a) ausgehend vom Vorderende (16) der kegelförmigen Kammer (12) innerhalb eines Innenraums der kegelförmigen Kammer (12) zu bilden.

7. Vorrichtung nach Anspruch 1, **dadurch gekennzeichnet, dass** die Kammer (12) eine äußere Kammer (80) ist, die ein Vorderende, ein Hinterende und eine gerippte äußere Struktur (86) aufweist, die so konfiguriert ist, das sie einen Durchgang eines Gebärmutterhalses durchquert.

8. Vorrichtung nach einem der Ansprüche 1-4 oder 7, **dadurch gekennzeichnet, dass** die rohrförmige Applikationskammer (30) im Durchmesser vom hintersten Ende (43) zur Öffnung (34) am Vorderende (30a) der Applikationskammer (30) hin schmäler wird; und
dass das Ende der rohrförmigen Applikationskammer (30) eine gekrümmte Spitze (91) umfasst, die einstückig mit der Applikationskammer (30) ist.

9. Vorrichtung nach einem der Ansprüche 1-4 oder 7, **dadurch gekennzeichnet, dass** sie eine gekrümmte Spitze (93) umfasst, die in einem Winkel (94) zwischen 18 und 30 Grad zu einer Mittelachse einer äußeren Kammer (80) gebogen ist.

## Revendications

1. Instrument (10) destiné à déposer des substances (11) dans l'utérus d'un mammifère, comprenant :
une chambre (12) ayant une extrémité antérieure conique (16), une extrémité postérieure (14), et configurée en vue de traverser un passage du col de l'utérus ;
une gaine (20) possédant une extrémité antérieure (24) et une extrémité postérieure (22), laquelle extrémité antérieure s'étend dans le sens axial à partir de l'extrémité postérieure (14) de la chambre (12) ; et
une chambre de dépôt (30) coulissante possédant une extrémité arquée (32) et destinée à s'étendre dans le sens axial entre la chambre conique (12) et une position située au-delà de l'extrémité antérieure (16) de la chambre conique,
dans lequel l'extrémité arquée (32) possède une ouverture (34) à son extrémité antérieure.

2. Instrument selon la revendication 1, **caractérisé en ce que** :
l'extrémité antérieure (16) conique de la chambre (12) se compose d'un matériau souple en polychlorure de vinyle (PVC) et possède une pluralité de perforations (36) qui s'étendent latéralement vers l'extrémité antérieure de la chambre conique et sont d'une longueur suffisante pour permettre le repliement de l'extrémité arquée (32) à l'intérieur ;
la chambre de dépôt (30) possède un repère visuel (44) situé sur une surface extérieure de la chambre de dépôt pour indiquer une orientation relative de l'extrémité arquée de la chambre de dépôt ;
l'extrémité arquée (32) de la chambre de dépôt (30) comprend en outre une partie plane (46), laquelle partie plane (46) est configurée de façon à buter sur une paroi distale de l'utérus afin de retarder l'insertion plus avant de la chambre de dépôt ; et
comprenant en outre un réceptacle à liquide (40) fixé à l'extrémité postérieure (22) de la gaine (20) et dans lequel l'extrémité postérieure de la gaine a en outre une forme tronconique (38) configurée de façon à pouvoir être introduite dans le réceptacle (40).

3. Instrument selon la revendication 1 ou 2, **caractérisé en ce que** l'ouverture (34) de l'extrémité arquée (32) a un rayon intérieur (48) et un rayon extérieur (52).

4. Instrument selon la revendication 3, **caractérisé en ce qu'**il comporte une partie curviligne surélevée (54) qui s'étend entre le rayon intérieur (48) et le rayon extérieur (52).

5. Instrument selon la revendication 1, **caractérisé en ce que** :
la chambre conique (12) possède une formation spiralée externe (18) configurée de façon à franchir le passage cervical ;
**en ce que** la gaine (20) possède une extrémité postérieure (38) tronconique qui s'étend dans le sens axial à partir de l'extrémité postérieure de la chambre conique ;
**en ce que** l'extrémité arquée (32) de la chambre de dépôt (30) possède une double courbure (32a, 32b) et une ouverture (34) pour permettre au sperme de s'écouler hors de la chambre de dépôt (30) pour entrer dans l'utérus ; et
**en ce qu'**une unité servant de contenant pour des embryons ou du sperme (40) est raccordée à la chambre de dépôt à l'extrémité postérieure (43) de la gaine (20) avec un adaptateur (56).

6. Instrument selon l'une quelconque des revendications précédentes, **caractérisé en ce qu'**une pluralité de perforations (36) s'étend latéralement de manière à créer des ailettes (36a) à partir de l'extrémité antérieure (16) de la chambre conique (12) dans un espace intérieur de la chambre conique (12).

7. Instrument selon la revendication 1, **caractérisé en ce que** la chambre (12) est une chambre extérieure (80) possédant une extrémité antérieure, une extrémité postérieure et une formation externe nervurée (86) configurée de manière à franchir un passage du col de l'utérus.

8. Instrument selon l'une quelconque des revendications 1-4 ou 7, **caractérisé en ce que** la chambre de dépôt (30) tubulaire diminue en diamètre de l'extrémité postérieure (43) à l'ouverture (34) de l'extrémité antérieure (30a) de la chambre de dépôt (30) ; et
**en ce que** l'extrémité de la chambre de dépôt (30) comprend une pointe recourbée (91) solidaire de la chambre de dépôt (30).

9. Instrument selon l'une quelconque des revendications 1-4 ou 7, **caractérisé en ce qu'**il comprend une pointe recourbée (93) qui se recourbe selon un angle (94) compris entre 18° et 30° par rapport à un axe central d'une chambre extérieure (80).
